Europäisches Patentamt

European Patent Office

Office européen des brevets

(11) Publication number: **0 313 527**
**A2**

# EUROPEAN PATENT APPLICATION

(21) Application number: **88830427.6**

(22) Date of filing: **20.10.88**

(51) Int. Cl.⁴: **A 61 L 2/16**
**A 61 L 2/18**

(30) Priority: **22.10.87 IT 366587**

(43) Date of publication of application:
**26.04.89 Bulletin 89/17**

(84) Designated Contracting States:
**CH DE ES FR GB GR IT LI SE**

(71) Applicant: **CASTELLINI S.p.A.**
**Via Saliceto, 22**
**I-40013 Castelmaggiore (Bologna) (IT)**

(72) Inventor: **Castellini, Franco**
**Via Bellinzona, 60**
**I-40135 Bologna (IT)**

(74) Representative: **Lanzoni, Luciano**
**c/o BUGNION S.p.A. Via Farini, 37**
**I-40124 Bologna (IT)**

(54) **A method for continuous sterilization of the waste pipelines of medical equipment or accessories, and a relative preparation.**

(57) The method is one whereby the waste pipelines of dental surgery and similar equipment can be sterilized continuously by exposing each line to the action of a slow-dissolving solid cleansing tablet comprising a sterilizer, a foam inhibitor and solubilizing and emulsifying agents. With such a tablet strategically located, water discharging through the line will dissolve and entrain the various substances continuously, carrying them along the length of the pipeline; in this way, organic matter is denatured and broken up, and the proliferation of microorganisms inhibited.

EP 0 313 527 A2

**Description**

**A method for continuous sterilization of the waste pipelines of medical equipment or accessories, and a relative preparation**

The invention relates to a method for continuous sterilization of the waste pipelines of medical equipment or accessories, and in particular, dental surgery equipment, also to a chemical preparation serving for the implementation of such a method. Medical equipment and accessories of the type in question must be subjected to sterilization in order to prevent the spread of infection from one patient to another, or from the practitioner to the patient; more precisely, sterilization signifies the forcible and substantially total elimination of bacteria from such equipment, pathogenic bacteria in particular.

This type of sterilization is especially important in the dental surgery, where every single session of treatment, even a simple inspection, necessarily involves the introduction of instruments into the mouth of the patient, hence into contact with a major vehicle of infection.

Currently, continuous sterilization (or at least disinfection) in modern dental surgery equipment is limited to the instruments proper, together with their handgrips and spray circuits; in addition to these items, the typical equipment column will also incorporate a suction system for the extraction of saliva, a spittle bowl, and a water tap from which to replenish a drinking glass used by the patient for oral rinsing purposes.

The suction system is connected to a canister by which extracted fluids are separated into gases and liquids, and in which waste matter accumulates. More exactly, it is in the canister that substances drawn in from the patient's mouth are collected, namely saliva, blood, drilling debris and coolant sprayed from rotary instruments such as the high speed drill.

A conventional suction system comprises at least one strainer, or filter element, installed on the canister inlet line and serving to trap the larger particles of solid matter.

In effect, the canister consists in an evacuated reservoir, and it is therefore necessary to ensure that the liquids accumulating internally of the enclosed space are prevented from foaming, as this will prevent the system from operating correctly.

Accordingly, the filter is lodged in an accessible housing so as to enable cleaning and removal, and create a place in which to position a tablet with foam inhibiting properties that dissolves gradually in the liquid by which it is invested.

Besides the canister and filter, the suction system also comprises an interchangeable cannula attached to one end of a flexible tube, say, in a simple snap fit, the remaining end of which is connected to the canister; the filter is located along this flexible line between the cannula and the canister. Sterilization of these conventional suction systems is effected periodically, normally at the end of each working day, by drawing a liquid disinfectant through the flexible tube and into the canister. A sterilization procedure of this type is beset by several limitations and drawbacks, foremost among which is that it is not continuous. The time lapse between one sterilization and the next, in fact, is such as to permit degeneration and decomposition of the organic matter contained in the canister, with the result that significant numbers of germs are allowed to collect in the enclosure, for example Lactobacillaceae and many other organisms found in the bacterial flora of the oral cavity, which are drawn into the suction circuit during treatment. With germs and bacillaceae thus accumulating in the canister together with putrefiable organic matter, the multiplication of infecting microorganisms is significantly favoured.

Matter collecting in the canister forms deposits and slime, often foul-smelling, and this further compounds the proliferation of microorganisms (on average, bacterial flora will double in quantity in some 30 minutes). As conventional non-continuous sterilization is able to slow up this proliferation only for a limited period, i.e. following intake of the liquid disinfectant, it is evident that one has only a limited and at best temporary effectiveness of sterilization in terms of eliminating bacteria; germs are thus able to continue multiplying, even reaching the cannula in some instances.

Another drawback of the conventional method stems from the brevity of the sterilizing action (which in many instances is no more than a disinfection, strictly considered); in effect, a limited quantity of liquid disinfectant is sent through the suction line for a limited period of time, and often will be unable to penetrate every single part of the pipeline with which the fouled liquids are brought into contact during the interval of time between one sterilization and the next.

A further drawback with conventional sterilization is due to the limited period of contact between the liquid disinfectant and the surfaces or components exposed thereto, given that the effectiveness of the disinfecting action is directly proportional to the period of exposure.

Poor sterilization constitutes a hazard not only for the patient, but for the medical practitioner too, as there must also be an effect on those parts subject to frequent handling occasioned by cleaning and replacement requirements. Such is the case, for example, with the cannula suction line filter; the very action of this component dictates that it must harbour the waste solids of largest dimensions, and these are among the major sources of bacteria, more especially pathogenic organisms.

Accordingly, the object of the present invention is to set forth a sterilization method that can be implemented continuously through the course of each working day.

The stated object is realized comprehensively with a method of sterilization as characterized in the appended claims, which consists in locating a solid chemical preparation in the waste pipelines of the equipment colum; the preparation envisaged and claimed is a slow-dissolving compound, comprising at least one sterilizing medium, a foam inhibitor (where necessary) and emulsifying and solubilizing agents.

One advantage afforded by the method disclosed is that of its effectiveness, gained by virtue of the fact that the solid sterilizer acts directly and continuously on the discharging liquid. The effect can be enhanced, moreover, by locating the solid sterilizer at the point of maximum pollution, i.e. where one has the greatest accumulation of debris and therefore the greatest build-up of bacteria, especially pathogenic. This aspect is particularly advantageous as regards sterilization of the one or more filters, as these can then be handled with greater peace of mind.

A further advantage of the method is the ease with which the canister can be cleaned; the sterilizer in fact performs a preventive action, discouraging the formation of hard deposits and slime internally of the canister through its capacity to denature (i.e. to inhibit decomposition and putrefaction) and/or break up organic substances.

Yet another advantage of the method disclosed is the facility it affords to effecting sterilization of the entire waste pipeline into which the solid preparation, a tablet, is introduced. More exactly, such a tablet can be positioned in the suction line at a point either coinciding with or immediately downstream of the cannula, so that the only stretch of the line not directly subject to the action of the sterilizer will be the cannula itself.

In any event, the cannula can be changed over with a freshly sterilized replacement following each session of treatment.

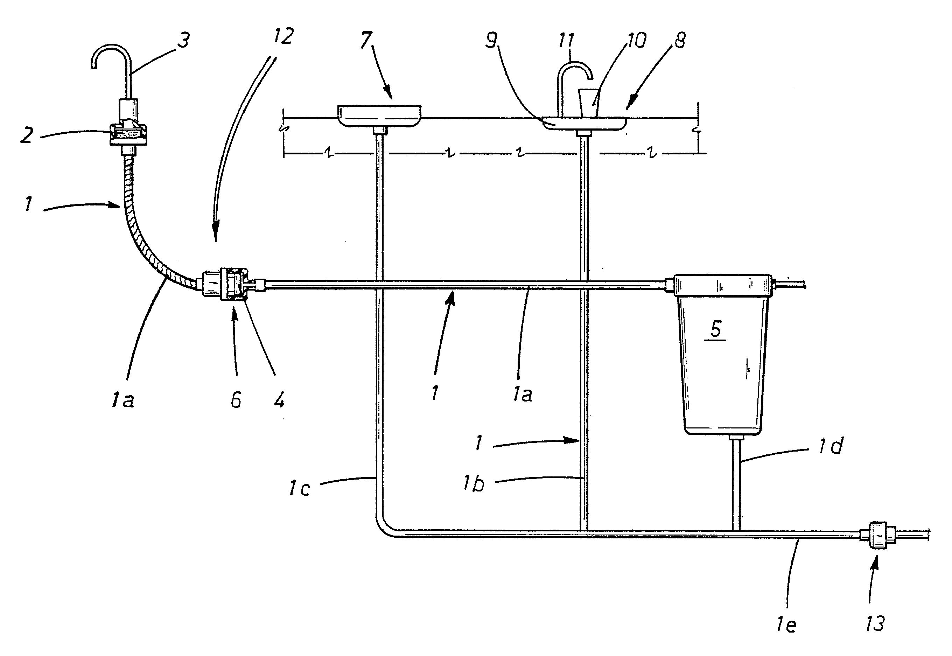

The invention will now be described in detail by way of example with the aid of the accompanying drawing, which illustrates a preferred arrangement of the waste pipelines in dental surgery equipment, as regards sterilization by adoption of the method and the preparation disclosed.

In the illustration of the equipment column, which is schematic, 7 denotes the spittle bowl, 8 denotes a stand, with a water tap, accommodating a drinking glass used by the patient for rising purposes, and 12 denotes the suction circuit, in its entirety.

The spittle bowl 7 appears as an open-topped basin, which is swilled by a relative supply of water. The rinse stand 8 consists essentially in a sunken cup, or well 9, that supports a drinking glass 10, and a water tap with an arched spout 11 that is directed down into the glass. The well 9 is provided with an outlet, connecting with a pipe denoted 1b, by way of which to drain off any water that may drip from the spout 11 when the drinking glass 10 is removed.

The suction circuit 12 is conventional, comprising an interchangeable cannula 3 fitted to one end of a flexible tube 1a; the remaining end of the tube is connected to a canister 5 in which extracted fluids separate into gas and liquid. Also connected into the line 1a is a filter housing 6 accommodating an element denoted 4.

The spittle bowl 7, rinse stand 8 and canister 5 all discharge by way of respective pipelines 1c, 1b and 1d into a main waste line 1e, and in most cases this line too will include a filtration unit 13. With reference to the drawing, the method according to the invention involves exposing the pipelines 1 of the surgery equipment column to the action of a solid and preferably slow-dissolving preparation 2, packaged in tablet form for example, designed to sterilize a liquid by which it is invested.

The tablet 2 may be located at any given point in the pipeline 1, though clearly enough, a point as near as possible to the source of polluton will be the most effective; the essential factor is that the tablet be correctly invested by the discharging liquid.

In the case of the spittle bowl 7 and the drinking glass stand 8, advantageous positions might be inside the basin, and the well 9, respectively. As regards the suction circuit 12, the tablet 2 will be positioned to best advantage immediately downstream of the cannula 3, in such a way that sterilization of the entire waste line 1a and 1d can be made continuous.

Other positions of good effect could be immediately upstream of the filter 4 and its housing 6, or of the canister 5, or alternatively, inside the second filter 13 on the main waste line.

Locating the tablet in any one of these positions, its action will be concentrated precisely where the polluting effect is greatest. It will be observed, in fact, that the filter 4 poses an obstruction to the discharging liquid flowing to the canister 5, and therefore occasions a certain build-up of the organic substances entrained, albeit less than that which occurs at the canister 5.

The preparation 2 according to the invention is a solid composition of sterilizing, emulsifying and solubilizing agents.

In the event that such a preparation 2 is utilized to sterilize the suction circuit, a foam inhibiting agent will also be incorporated (indispensable for correction operation of the canister).

The proportions of the various components making up the tablet may vary according to its location, and to the conditions in the pipeline to be sterilized -i.e. to the rate of flow and the velocity of the liquid by which the tablet is invested.

In a preferred preparation, the concentration of the various components will be such as to maintain the pH value of a discharging liquid within neutral limits, that is, between 6 and 8.

The sterilizing agent, which is the indispensable component, might be a stabilized chlorine-bearing substance, i.e. capable of releasing and generating active chlorine in water, or a phenolic compound, an iodine-bearing substance, a quaternary ammonium or nitrogen compound, a peroxide, a persalt, etc... A preferred substance will be one selected from the chlorine-bearing group which includes, for example, chlorine isocyanides and their salts, chloramines and the like; in particular, optimum results have been obtained at experimental level using sodium dichloroisocyanate.

Accordingly, a preferred composition would comprise sodium dichloroisocyanate (15...20%), providing the stabilized chlorine-bearing agent, a silicone based foam inhibitor (05...10%), and a polyglycol as the solubilizing agent and vehicle (55...67%).

Two silicones can be used in formulating the foam inhibitor, one an emulsion (5% approx), the other a solid or a powder (5% approx.).

The basic composition is supplemented further with a conditioning and stabilizing agent (5...15%) that assists the solubilizing action between unctuous matter and water, and thus enhances the sterilizing action of the active chlorine released from the preparation; polysorbate ethoxylate or anhydrous sodium metasilicate would be suitable conditioners. The finished blend will also be scented by addition of a substance such as eucalyptus.

The following page shows two preferred compositions according to the invention, A and B, that have been obtained from practical experiment:

Composition A

| | |
|---|---|
| - polyethylene glycol | 62.00% |
| - Tween 80 (surface-active emulsion) | 8.05% |
| - sodium stearate | 2.40% |
| - magnesium stearate | 3.20% |
| - sodium dichloroisocyanate | 16.30% |
| - oil of silicone | 6.45% |
| - scent (eucalyptus) | 1.60% |
| | 100.00% |

Composition B

| | |
|---|---|
| - polyethylene glycol | 59.00% |
| - anhydrous sodium metasilicate | 10.00% |
| - sodium stearate | 1.00% |
| - sodium dichloroisocyanate | 20.00% |
| - silicone emulsion | 8.00% |
| - scent (eucalyptus) | 2.00% |
| | 100.00% |

It will be clear enough that, in dental surgery equipment arranged for implementation of the method disclosed, discharging liquids will wash over the tablet 2 continuously, dissolving it in similarly continuous fashion. The components are entrained steadily into the flowing liquid, and thus perform their sterilizing action (and their foam inhibiting action, if so formulated) both on the liquid itself and on the surfaces of the waste pipeline 1 bathed by the liquid. Emerging ultimately from the line 1 individually sterilized, the liquid and the fully dissolved components of the tablet 2 are directed into the main waste line 1e.

Thus, the preparation 2 becomes effective from the moment it is invested by the discharging liquid, and as regards cleanliness of the suction system, maximum effect is obtained at the earliest possible moment, prior to the entry of the liquid into the canister 5; in effect, an early break-up of organic matter will prevent a heavy accumulation of scale and slime in the canister 5, and thus avoid the proliferation of microorganisms.

The most significant advantage of the method and the preparation disclosed is that of the complete and continuous sterilization effected on all waste pipelines 1 downstream of the tablet 2 for as long as the tablet continues to dissolve.

A further advantage is that of the sure efficiency of the sterilizing action at all points reached by the treated liquid. Not only do fouled liquids no longer carry polluting substances with them; by contrast, they are charged with components capable of attacking and destroying such pollutants.

The foregoing specification implies no limitation at any level; for example, the stabilized chlorine-bearing substance indicated might be replaced by succinchlorimide or trichloromelamine.

**Claims**

1) A method for continuous sterilization of the waste pipelines of medical equipment or accessories, in particular dental surgery equipment,
characterized
in that it consists in inserting a preparation (2) into the waste pipelines (1) of the equipment in a position such as will cause it to be invested by a discharging liquid; and
in that the preparation is a slow-dissolving solid composition comprising at least one sterilizing agent.

2) A method for continuous sterilization of the waste pipelines of medical equipment or accessories, in particular dental surgery equipment,
characterized
in that it consists in inserting a preparation (2) into the waste pipelines (1) of the equipment in a position such as will cause it to be invested by a discharging liquid; and
in that the preparation is a slow-dissolving solid composition comprising at least one sterilizing agent and at least one foam-inhibiting agent.

3) A method for continuous sterilization of the waste pipelines of medical equipment or accessories, in particular dental surgery equipment,
characterized
in that it consists in inserting a preparation (2) into the waste pipelines (1) of the equipment in a position such as will cause it to be invested by a discharging liquid; and
in that the preparation is a slow-dissolving solid composition comprising at least one sterilizing agent, at least one foam-inhibiting agent, and solubilizing and emulsifying agents that serve to condition the chemistry of a discharging liquid.

4) A method as in claim 1, 2 or 3 for continuous sterilization of the waste pipelines of medical equipment or accessories, in particular dental surgery equipment incorporating waste pipelines that connect a cannula (3), a rinse stand and water tap assembly (8), a spittle bowl (7) and a fluid separation canister (5) with a main waste outlet, and comprising filters (4, 13) installed on the canister inlet and the main waste outlet lines, respectively, wherein the preparation (2) is located at least to coincide with the cannula (3).

5) A method as in claim 1, 2 or 3 for continuous sterilization of the waste pipelines of medical equipment or accessories, in particular dental surgery equipment incorporating waste pipelines that connect a cannula (3), a rinse stand and water tap assembly (8), a spittle bowl (7) and a fluid separation canister (5) with a main waste outlet, and comprising filters (4, 13) installed on the canister inlet and the main waste outlet lines, respectively, wherein the preparation (2) is located at least to coincide with the canister inlet filter (4).

6) A method as in claim 1, 2 or 3 for continuous sterilization of the waste pipelines of medical equipment or accessories, in particular dental surgery equipment incorporating waste pipelines that connect a cannula (3), a rinse stand and water tap assembly (8), a spittle bowl (7) and a fluid separation canister (5) with a main waste outlet, and comprising filters (4, 13) installed on the canister inlet and the main waste outlet lines, respectively, wherein the preparation (2) is located at least to coincide with the canister (5).

7) A method as in claim 1, 2 or 3 for continuous sterilization of the waste pipelines of medical equipment or accessories, in particular dental surgery equipment incorporating waste pipelines that connect a cannula (3), a rinse stand and water tap assembly (8), a spittle bowl (7) and a fluid separation canister (5) with a main waste outlet, and comprising filters (4, 13) installed on the canister inlet and the main waste outlet lines, respectively, wherein the preparation (2) is located at least to coincide with the spittle bowl (7).

8) A method as in claim 1, 2 or 3 for continuous sterilization of the waste pipelines of medical equipment or accessories, in particular dental surgery equipment incorporating waste pipelines that connect a cannula (3), a rinse stand and water tap assembly (8), a spittle bowl (7) and a fluid separation canister (5) with a main waste outlet, and comprising filters (4, 13) installed on the canister inlet and the main waste outlet lines, respectively, wherein the preparation (2) is located at least to coincide with the rinse stand and water tap assembly (8).

9) A method as in claim 1, 2 or 3 for continuous sterilization of the waste pipelines of medical equipment or accessories, in particular dental surgery equipment incorporating waste pipelines that connect a cannula (3), a rinse stand and water tap assembly (8), a spittle bowl (7) and a fluid separation canister (5) with a main waste outlet, and comprising filters (4, 13) installed on the canister inlet and the main waste outlet lines, respectively, wherein the preparation (2) is located at least to coincide with the main waste outlet filter (4).

10) A preparation for continuous sterilization of the waste pipelines of medical equipment or accessories in particular dental surgery equipment,
characterized
in that it consists in a slowly and continuously dissolving solid composition comprising at least one sterilizing agent formulated in combination with emulsifying and solubilizing agents that are designed to ensure stability and solubility of the solid when invested by a discharging liquid.

11) A preparation as in claim 10, wherein the solid composition further comprises at least one foam-inhibiting agent.

12) A preparation as in claim 10, wherein the solid composition further comprises at least one foam-inhibiting agent, together with solubilizing agents, emulsifying agents, and agents serving to condition the chemistry of the discharging liquid.

13) A preparation as in claim 10 or 12, wherein the sterilizing agent is sodium dichloroisocynate.

14) A preparation as in claim 12, wherein the solid composition comprises: 15...20% sodium dichloroisocyanate, functioning as a stabilized chlorine-bearing substance; 05...10% silicone, functioning as a foam inhibiting agent; 55...67% polyglycol, functioning as a solubilizing agent and vehicle; 5...15% polysorbate ethoxylate or anhydrous sodium metasilicate, functioning as a conditioner and stabilizer that serves to assist the solubilizing action between unctuous matter and water, thereby enhancing the sterilizing action of the active chlorine released from the composition; and a remaining percentage of eucalyptus serving as a scent.

15) A preparation as in claim 14, comprising:

- polyethylene glycol
    62.00%
- Tween 80 (surface active emulsion)
    8.05%
- sodium stearate
    2.40%
- magnesium stearate
    3.20%
- sodium dichloroisocyanate
    16.30%
- oil of silicone
    6.45%
- scent (eucalyptus)
    1.60%

16) A preparation as in claim 14, comprising:

| - polyethylene glycol | 59.00% |
| - anhydrous sodium metasilicate | 10.00% |
| - sodium stearate | 1.00% |
| - sodium dichloroisocyanate | 20.00% |
| - silicone emulsion | 8.00% |
| - scent (eucalyptus) | 2.00% |